(19) Europäisches Patentamt
European Patent Office
Office européen des brevets

(11) EP 1 607 409 A1

(12) # DEMANDE DE BREVET EUROPEEN

(43) Date de publication:
**21.12.2005 Bulletin 2005/51**

(51) Int Cl.[7]: **C08F 2/38**

(21) Numéro de dépôt: **05291040.3**

(22) Date de dépôt: **13.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **15.06.2004 FR 0451182**

(71) Demandeur: **L'OREAL**
**75008 Paris (FR)**

(72) Inventeur: **Farcet, Céline**
**75012 Paris (FR)**

(74) Mandataire: **Dodin, Catherine**
**L'OREAL - D.I.P.I.**
**25-29 Quai Aulagnier**
**92600 Asnières (FR)**

(54) **Copolymère fonctionnalisé par un atome d'iode composition le comprenant et procédé de traitement**

(57) La présente demande concerne des nouveaux copolymères fonctionnalisés par un atome d'iode, et comprenant au moins deux monomères distincts, l'un étant choisi parmi les monomères de type soluble et l'autre étant choisi parmi les monomères de type insoluble.

L'invention concerne également une composition cosmétique ou dermatologique comprenant, dans un milieu physiologiquement acceptable, au moins un copolymère tel que défini ci-dessus.

L'invention concerne encore un procédé cosmétique de maquillage ou de soin des matières kératiniques, employant ladite composition.

EP 1 607 409 A1

## Description

La présente invention a trait à de nouveaux copolymères ainsi qu'aux compositions notamment cosmétiques, pharmaceutiques ou dermatologiques topiques les comprenant.

Dans le domaine de la cosmétique, on cherche souvent à disposer de compositions permettant d'obtenir un dépôt notamment adhésif ou filmogène, sur les matières kératiniques considérées, telles que les cheveux, la peau, les cils ou les ongles. En particulier, ces compositions peuvent apporter de la couleur (compositions de maquillage ou de coloration capillaire), de la brillance ou de la matité (compositions de soin ou de maquillage de la peau), des propriétés physiques telles que de la mise en forme (compositions capillaires notamment de coiffage), des propriétés de soin ou de protection (compositions de soin, par exemple d'hydratation ou de protection UV). On recherche généralement une bonne rémanence et tenue dans le temps du dépôt cosmétique, ainsi qu'une bonne adhésion sur le support. En particulier, il est souhaitable que ce dépôt puisse résister aux agressions mécaniques telles que frottements, transferts par contact d'un autre objet; à l'eau, à la sueur, aux larmes, à la pluie, au sébum et aux huiles. Ceci est particulièrement vrai en maquillage, notamment dans le domaine des rouge à lèvres où l'on recherche la tenue prolongée de la couleur et de la brillance et le non transfert de la couleur; dans le domaine des fonds de teint, fards à paupières et poudres où l'on recherche la tenue de la couleur apportée, en maintenant le plus longtemps possible la matité de la teinte initiale malgré la sécrétion de sébum et de sueur, ainsi que le non transfert. En outre, les compositions de maquillage doivent être confortables à porter et ne pas présenter une texture trop collante.
Pour concilier l'ensemble de ces propriétés, souvent antinomiques, au sein d'une même composition, on utilise généralement un mélange de plusieurs polymères, de nature chimiques très différentes, chaque polymère apportant une des caractéristiques souhaitées. Toutefois, l'emploi d'un mélange de polymères ayant des natures chimiques différentes, pas forcément compatibles, peut générer des problèmes de démixtion au sein de la composition.

L'utilisation de polymères statistiques, par exemple de polymères acryliques conventionnels obtenus par polymérisation radicalaire classique par mélange statistique de monomères, ne permet pas de résoudre ces problèmes de manière satisfaisante. En effet, les polymères statistiques connus dans l'art antérieur présentent une dispersité en composition des chaînes polymériques, ce qui conduit également à une démixtion des polymères au sein de la formule.

On connaît, notamment par le brevet US5,807,937, un procédé dit ATRP qui permet de préparer des polymères particuliers qui peuvent être fonctionnalisés à leur extrémité, notamment par un atome de brome ou de chlore; ce procédé nécessite toutefois l'emploi de catalyseurs métalliques, qui peuvent rester sous forme de trace dans les polymères ainsi préparés. Dans le cas d'applications cosmétiques de ces polymères, la présence de tels atomes peut éventuellement poser problème. De plus, ces atomes ne sont pas toujours suffisamment labiles pour que leur remplacement par des fonctions plus recherchées soit effectué aisément.
On connaît par ailleurs, par le document WO99/20659, des polymères fonctionnalisés à leur extrémité et obtenus par transfert dégénératif (TD). La polymérisation radicalaire contrôlée par transfert dégénératif (TD) permet la synthèse de (co)polymères fonctionnalisés dont la masse molaire et l'architecture sont contrôlées. Toutefois, il est essentiellement décrit des homopolymères préparés par cette technique.

La présente invention a pour but de pallier les inconvénients de l'art antérieur en proposant de nouveaux copolymères particuliers, fonctionnalisés à au moins une de leur extrémité par un atome suffisamment labile pour être aisément remplacé, et acceptable cosmétiquement.
Par ailleurs, les copolymères selon l'invention peuvent permettre d'éviter les problèmes de demixtion au sein de la formule tout en permettant d'apporter les propriétés cosmétiques recherchées.

Un objet de la présente invention est un copolymère fonctionnalisé à au moins l'une de ses extrémités par un atome d'iode, et comprenant au moins deux monomères distincts choisis l'un parmi les monomères de type soluble et l'autre parmi les monomères de type insoluble, tels que ci-après définis.

Un autre objet de l'invention est un copolymère susceptible d'être obtenu par polymérisation radicalaire contrôlée par transfert dégénératif à l'atome d'iode :

- d'un amorceur radicalaire,
- d'un agent de transfert comprenant au moins un atome d'iode, et
- d'au moins deux monomères distincts choisis l'un parmi les monomères de type soluble et l'autre parmi les monomères de type insoluble, tels que ci-après définis

Un autre objet de l'invention est une composition cosmétique ou dermatologique comprenant, dans un milieu physiologiquement acceptable, au moins un copolymère tel que défini ci-dessus.

Les copolymères selon l'invention présentent l'avantage, au moins pour une partie d'entre eux, de pouvoir facilement être mis en oeuvre dans les milieux cosmétiques organiques, notamment dans les milieux solvants et/ou huileux cosmétiques, tout en conservant des propriétés rhéologiques intéressantes.

En particulier, les copolymères selon l'invention présentent une bonne liposolubilité dans les solvants et/ou les huiles cosmétiques.

On rappelle que par "polymère soluble", on entend que le polymère ne forme pas de précipité dans le solvant. Avantageusement, le copolymère selon l'invention est soluble à une concentration d'au moins 3% en poids, dans l'isododécane, à 25°C, 1 atm., de préférence à une concentration d'au moins 5% en poids, voire d'au moins 10% en poids.

Les copolymères selon l'invention peuvent se présenter sous diverses formes, notamment sous forme de polymères séquencés (ou polymère blocs) ou bien sous forme de polymères à gradient. De préférence, le copolymère selon l'invention se présente sous la forme d'un polymère à gradient, ce qui permet de le véhiculer à un taux élevé, dans les compositions cosmétiques selon l'invention.

Dans un copolymère à gradient, la composition au sein de la chaîne de polymère suit un gradient de composition. De préférence, le copolymère à gradient selon l'invention présente une faible polydispersité en composition: toutes les chaînes de copolymères ont une composition (c'est-à-dire un enchaînement de monomères) approximativement analogue et sont donc homogènes en composition. Comme toutes les chaînes présentent quasiment les mêmes structures, elles sont compatibles entre elles; il en résulte que les compositions cosmétiques comprenant ces copolymères ne présentent pas les inconvénients et limitations des compositions de l'art antérieur.

Lorsque le copolymère selon l'invention est linéaire et à gradient, il peut être représenté, de façon schématique, par la formule suivante :

$$F\text{-}[M_1M_2]_{gradient}\text{ - I}$$

dans laquelle F représente un reste soit issu de l'agent de transfert (le radical R défini ci-après), soit issu de l'amorceur; I étant l'atome d'iode et $M_1$ et $M_2$ étant les monomères ou mélanges de monomères.

Les copolymères selon l'invention peuvent également se présenter sous forme de polymère séquencé, c'est-à-dire de polymère comprenant au moins 2 séquences successives distinctes, c'est-à-dire de natures chimiques différentes.

Chaque séquence, ou bloc, du copolymère selon l'invention est issue d'un type de monomère ou de plusieurs types de monomères différents. Cela signifie que chaque séquence peut être elle-même constituée d'un homopolymère ou d'un copolymère; ce copolymère constituant la séquence pouvant être à son tour statistique ou alterné ou à gradient; la répartition des monomères au sein de chaque séquence peut donc être aléatoire ou contrôlée selon la nature et/ou la réactivité des monomères.

Lorsque le copolymère selon l'invention est séquencé, il peut s'agir d'un polymère dibloc du type AB; ou bien d'un polymère tribloc par exemple du type ABA, BAB, ABC avec C différent de A et B; ou encore de polymères multiblocs ayant plus de trois séquences, par exemple du type (AB)n, (ABA)n, (BAB)n, (ABC)n, ABCD, avec A, B, C et D de nature chimique différente. De préférence, le copolymère selon l'invention comporte au moins 2 séquences successives,

ou bien 3 séquences successives, deux séquences successives étant différentes : il peut être par exemple de type AB, ABA ou ABC.

Lorsque le copolymère selon l'invention est linéaire et séquencé, il peut être représenté, de façon schématique, par la formule suivante : $F\text{-}[(M_1)_n\text{-}(M_2)_m]$ - I ,

dans laquelle n et m sont des entiers strictement supérieurs à 1.

Les copolymères selon l'invention peuvent également se présenter sous forme de polymères étoiles, chaque branche de l'étoile pouvant être à gradient ou à blocs. On obtient des polymères étoiles lorsque l'agent de transfert est polyfonctionnel et compte au moins trois atomes d'iode.

Dans le cas d'un copolymère en étoile, le copolymère peut être représenté d'une manière générale par la formule $F\text{-}[M_1M_2\text{-}I]_{n'}$ avec $M_1$ et $M_2$ différents, représentant un monomère seul ou un mélange de monomères, et étant agencés selon un gradient ($F\text{-}[(M_1M_2)_{grad}\text{-}I]_{n'}$) ou pouvant former des séquences ou blocs distincts ($F\text{-}[(M_1)_n(M_2)_m\text{-}I]_{n'}$), et n' étant le nombre de branches de l'étoile et étant un entier strictement supérieur à 2, par exemple compris entre 3 et 8.

Lorsque n' = 2, le polymère obtenu est linéaire et difonctionnel.

Le copolymère selon l'invention peut également être un copolymère greffé, le squelette dudit polymère pouvant être à gradient ou à blocs. On obtient des polymères greffés lorsqu'au moins un des monomères (M1 et/ou M2) est un macromonomère tel que ci-après défini.

D'une manière générale, les copolymères selon l'invention peuvent être séquencés ou en gradient, selon une structure de chaîne linéaire, greffée ou en étoile.

De préférence, le copolymère selon l'invention est un copolymère linéaire, à gradient ou séquencé.

De préférence, la masse moléculaire moyenne en nombre du copolymère selon l'invention est comprise entre 2000 g/mol et 1 000 000 g/mol, notamment entre 3000 g/mol et 500 000 g/mol, et encore mieux entre 4000 g/mol et 200 000 g/mol, voire 5000 g/mol et 50 000 g/mol.

On détermine la masse moléculaire moyenne en nombre (Mn) par chromatographie liquide par perméation de gel (GPC), éluant THF, courbe d'étalonnage établie avec des étalons de polystyrène linéaire, détecteur réfractométrique.

Les copolymères selon l'invention sont obtenus par polymérisation radicalaire contrôlée par transfert dégénératif à l'atome d'iode.

La polymérisation radicalaire contrôlée désigne des polymérisations pour lesquelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. L'imperfection de ce mode de polymérisation réside dans le fait que lorsque les concentrations en radicaux libres deviennent importantes par rapport à la concentration en monomère, les réactions secondaires redeviennent déterminantes et tendent à élargir la distribution des masses.

Grâce à ce mode de polymérisation, les chaînes polymériques des copolymères à gradient selon l'invention croissent simultanément et donc incorporent à chaque instant les mêmes ratio de co-monomères. Toutes les chaînes possèdent donc les mêmes structures ou des structures proches, d'où une faible dispersité en composition. Ces chaînes possèdent également un indice de polydispersité en masse faible. Les copolymères à gradient sont des copolymères présentant une évolution du ratio des différents monomères tout au long de la chaîne. La distribution dans les chaînes polymériques des co-monomères dépend de l'évolution pendant la synthèse des concentrations relatives des co-monomères. Les copolymères à gradient selon l'invention comprennent au moins deux monomères différents dont la concentration le long de la chaîne polymérique change graduellement et de façon systématique et prédictible. Ceci signifie que toutes les chaînes polymériques ont au moins un monomère Mi pour lequel, quelque soit la position normalisée x sur la chaîne polymérique, il y a une probabilité non nulle de retrouver ce monomère Mi le long de la chaîne. Une des caractéristiques permettant de définir les copolymères à gradient est le fait qu'à tout instant de la polymérisation, toutes les chaînes sont soumises à la présence de l'ensemble de tous les monomères. Ainsi, dans le milieu réactionnel, la concentration de chaque monomère est toujours non nulle, à tout instant de la polymérisation.

Ceci permet de différencier les copolymères à gradient des polymères blocs

dans lesquels l'évolution des monomères le long de la chaîne polymérique n'est pas systématique : par exemple pour un dibloc AB, au sein de la séquence A, la concentration en l'autre monomère B est toujours nulle.

Dans le cas des polymères statistiques, l'évolution des monomères le long de la chaîne polymérique ne sera pas non plus graduelle, systématique et prédictible.

De plus, parmi les copolymères à gradient, on peut distinguer les copolymères à gradient naturel, et les copolymères à gradient artificiel.

Un copolymère à gradient naturel est un copolymère à gradient synthétisé en batch à partir d'un mélange initial des co-monomères. La distribution dans la chaîne des divers monomères suit une loi déduite de la réactivité relative et des concentrations initiales de monomères. Ces copolymères constituent la classe la plus simple de copolymères à gradient car c'est le mélange initial qui définit la propriété finale de produit.

Un copolymère à gradient artificiel est un copolymère dont on fait varier la concentration en monomères durant la synthèse par un artifice de procédé. Dans ce cas, on passe d'un mélange de monomères à un autre dans la chaîne du fait d'un changement subit et brusque des monomères dans le milieu réactionnel (stripping du premier mélange ou addition d'au moins un nouveau monomère). Il peut même y avoir une disparition nette d'un ou plusieurs des monomères au profit d'un ou plusieurs autres.

Par ailleurs, dans un copolymère à gradient, la distribution des compositions des chaînes est étroite. En particulier il n'existe pas de recouvrement entre le pic du copolymère à gradient et ceux des homopolymères respectifs. Ceci signifie que le matériau obtenu en gradient est constitué de chaînes polymères de même composition alors qu'en polymérisation statistique classique, différentes sortes de chaîne coexistent, y compris celles des homopolymères respectifs. Les facteurs déterminant le gradient sont d'une part les coefficients de réactivité relatifs de chaque monomère (appelé $r_i$ pour le monomère Mi) qui dépendent principalement du type de procédé de synthèse employé (homogène, dispersé) et des solvants, et d'autre part les concentrations initiales de chacun des monomères, ainsi que les ajouts éventuels de monomères au cours de la polymérisation.

Les copolymères selon l'invention sont de préférence préparés selon un procédé dit de transfert dégénératif (ou degenerative iodine transfer ou DIT). Ce procédé permet de former des copolymères fonctionnalisés, en particulier mono-

ou multi-fonctionnalisés, à au moins l'une de leurs extrémités, par un atome d'iode. Ce procédé est notamment décrit dans la demande WO99/20659.

Selon ce procédé, on fait réagir les monomères choisis, avec un amorceur de polymérisation en présence d'un agent de transfert iodé.

L'amorceur de polymérisation peut être tout amorceur connu de l'homme de l'art pour son emploi dans les procédés de polymérisation radicalaire, et notamment des composés de type azoïque et notamment l'azobisisobutyronitrile;

ou de type peroxyde tels que les hydroperoxydes ou les peroxydes organiques ayant 6-30 atomes de carbone, notamment le peroxyde de benzoyle ou de didécanoyle; ou encore des couples rédox, des peresters, des percarbonates ou des persulfates.

De préférence, on choisit l'amorceur parmi les peroxydes organiques comprenant de 8 à 30 atomes de carbone, tels que le peroxyde de didécanoyle notamment commercialisé sous la référence Perkadox® SE-1 0 par la société Akzo Nobel.

On peut également citer comme amorceur le 2,5-bis(2-ethylhexanoylperoxy)-2,5dimethylhexane (Trigonox 141 de la société Akzo Nobel) et le tert-butyl peroxy-2-ethylhexanoate (Trigonox 21 S de la société Akzo Nobel).

L'agent de transfert peut être représenté par la formule R-I, dans laquelle R est un radical alkyle, linéaire, ramifié ou cyclique, saturé ou insaturé, comprenant 1 à 30 atomes de carbone, et pouvant comprendre en outre 1 à 4 atomes d'iode supplémentaires, et/ou un ou plusieurs atomes de fluor, et/ou une ou plusieurs fonctions choisies parmi CN, COOH.

De préférence, R est choisi parmi les alkyles ayant 1 à 6 atomes de carbone, comprenant éventuellement un ou plusieurs atomes d'halogène, notamment de fluor et/ou une fonction CN; en particulier R peut être un alkyle en C1-C6 perfluoré ou un alkyl en C1-C6 portant une fonction CN.

On peut notamment citer comme agent de transfert, le iodo-1-perfluorohexane, l'iodoacétonitrile ($ICH_2$-CN), le iodo-1-méthane, le diiodo-méthane, l'iodoforme
ou triiodo-méthane, l'iodo-1-perfluoroisopropane, le diiodoperfluorohexane, l'iodo-1-phenylethane, l'iodo-1-propane, l'iodo-1-isopropane, l'iodo-1-phényle, le 1,4-diiodo-phényle et l'iodo-1-tertio-butane.

Par ailleurs, l'agent de transfert peut être macromoléculaire et se présenter sous forme d'un polymère, homopolymère ou copolymère, obtenu par une étape préalable de polymérisation par transfert dégénératif à l'atome d'iode, et donc fonctionnalisé par au moins un atome d'iode.

Ceci est notamment le cas lorsque l'on souhaite préparer des copolymères-blocs.

La proportion molaire, r, entre l'agent de transfert et l'amorceur peut varier entre 0,1 et 20 avec une préférence entre 1 et 10 et plus précisément entre 2 et 5.

La proportion molaire, DP, entre l'ensemble des monomères et l'agent de transfert est de préférence supérieure à 10, notamment comprise entre 50 et 1000, et plus précisément comprise entre 100 et 500.

D'une manière schématique, les copolymères selon l'invention peuvent être préparés par l'homme du métier suivant le mode opératoire suivant :

1/ On prépare un mélange des différents monomères, éventuellement dans un solvant, de préférence dans un réacteur et sous agitation. On ajoute l'amorceur de polymérisation radicalaire et l'agent de transfert. Le mélange est mis de préférence sous atmosphère de gaz inerte par rapport à une polymérisation radicalaire tel que l'azote ou l'argon.

Comme solvant éventuel de polymérisation, on peut choisir le milieu solvant de la composition, par exemple des solvants et/ou des huiles cosmétiques tels que définis ci-après. En particulier, on peut choisir le solvant de polymérisation parmi les acétates d'alkyle tels que l'acétate de butyle ou l'acétate d'éthyle, des solvants aromatiques tels que le toluène ou des alcanes tels que l'isododécane, l'heptane ou l'isohexadécane.

On choisit préférentiellement un solvant de polymérisation dans lequel les monomères et le polymère résultant sont solubles.

2/ On porte le mélange sous agitation à la température de polymérisation souhaitée. Cette température est de préférence choisie dans une gamme allant de 20°C à 120°C, de préférence de 40°C à 90°C.

Le choix de la température de polymérisation est de préférence optimisé en fonction de la composition chimique du mélange de monomères.

3/ Le milieu de polymérisation est éventuellement modifié pendant la polymérisation, avant d'atteindre 90% de conversion des monomère initiaux, par ajout supplémentaire d'un ou plusieurs monomères, notamment du mélange initial. Cet ajout peut être réalisé de différentes manières, pouvant aller de l'addition brutale en une seule fois à l'addition continue sur la durée totale de la polymérisation.

4/ on arrête la polymérisation lorsque le taux de conversion souhaité est atteint. De cette conversion dépend la composition globale du copolymère. De préférence, on arrête la polymérisation après avoir atteint au moins 50% de conversion, notamment au moins 60%, préférentiellement après avoir atteint au moins 90% de conversion.

5/ Les éventuels monomères résiduels peuvent être éliminés par toute méthode connue, telle que par évaporation, ou par ajout d'une quantité d'initiateur classique de polymérisation tel que les dérivés peroxydiques ou azoïques.

6/ Lorsque l'on souhaite préparer des copolymères-blocs, on peut dans une étape supplémentaire ajouter alors

le ou les monomères additionnels, et éventuellement l'agent de transfert et l'amorceur, pour former un second bloc.

Le copolymère selon l'invention peut être obtenu par polymérisation radicalaire en masse ou en solution en milieu organique. Les monomères peuvent être ajoutés simultanément, en batch, en semi-continu ou consécutivement.

Pour une polymérisation en batch, on mélange le solvant éventuel, les monomères et l'amorceur dans un réacteur et on chauffe à la température requise.

Pour une polymérisation en semi-continu, on introduit dans un réacteur, tout ou partie du solvant éventuel, une partie des monomères, notamment 1 à 20% en poids du poids total de monomère, une partie de l'amorceur, notamment 1 à 20% en poids du poids total d'amorceur, éventuellement une partie de l'agent de transfert, notamment 0,1 à 20% en poids du poids total d'agent de transfert, et on chauffe à la température requise. Le reste du solvant, des monomères et de l'amorceur est introduit par une coulée, en cours de polymérisation. Les constituants restants peuvent être introduits par des coulées distinctes ou identiques, simultanées ou non.

Lorsque l'on souhaite préparer des copolymères à blocs ou séquencés, on emploie de préférence un procédé dans lequel le premier bloc suit un procédé de polymérisation (batch ou semi-continu). A l'issu de la polymérisation du premier monomère (ou mélange de premiers monomères), le second monomère (ou mélange de seconds monomères) est ajouté en batch ou semi-continu.

Lorsque l'on souhaite préparer des copolymères à gradient de composition, on introduit de préférence les monomères soit en batch, soit en semi-continu. Dans ce cas, la polymérisation d'un premier monomère (ou mélange de premiers monomères) est débutée et le second monomère (ou mélange de seconds monomères) est ajouté simultanément en batch ou semi-continu avant que la polymérisation du premier monomère ne soit achevée.

Le copolymère selon l'invention comprend au final au moins deux monomères distincts, au moins un étant choisi parmi les monomères dits "solubles" et au moins un autre étant choisi parmi les monomères dits "insolubles".

On entend par "monomère soluble", tout monomère dont l'homopolymère est soluble, c'est-à-dire ne forme pas de précipité dans le solvant, à une concentration d'au moins 3% en poids, dans l'isododécane, à 25°C, 1 atm. De préférence, on choisit le ou les monomères solubles parmi les monomères dont l'homopolymère est soluble à une concentration d'au moins 5% en poids, voire 10% en poids, dans l'isododécane, à 25°C, 1 atm.

On entend par "monomère insoluble", tout monomère dont l'homopolymère n'est pas soluble à une concentration d'au moins 3% en poids, dans l'isododécane, à 25°C, 1 atm.

Les monomères solubles peuvent en particulier être choisis parmi les monomères suivants, seuls ou en mélange, ainsi que leurs sels;

- (i) les (méth)acrylates de formule $CH_2=CHCOOR$ ou $CH_2=C(CH_3)COOR$ dans laquelle R représente :

  - un groupe alkyle linéaire ou ramifié, comprenant 8 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène ou un groupe alkyle linéaire ou ramifié, en C1 à C6 ou un groupe phényle;
  - un groupe cycloalkyle en C8 à C12,

  notamment R peut être un groupe octyle, décyle, lauryle, isooctyle, isodécyle, dodécyle, t-butylcyclohexyle, stéaryle, éthyl-2-hexyle, isobomyle, béhényle;

- (ii) le méthacrylate de tertiobutyle et l'acrylate d'isobutyle;

- (iii) les (méth)acrylamides de formule $CH_2=CHCONR_4R_5$ ou $CH_2=C(CH_3)CONR_4R_5$ dans laquelle :

  - R4 représente un atome d'hydrogène ou un groupe alkyle, linéaire ou ramifié, comportant de 1 à 6 atomes de carbone; de préférence, R4 est choisi parmi: hydrogène, méthyle, éthyle, propyle, isopropyle, n-butyle, isobutyle, tertiobutyle, hexyle, isohexyle, cyclohexyle;
  - R5 représente un groupe alkyle, linéaire ou ramifié, comportant de 8 à 18 atomes de carbone;

  de préférence, R5 est choisi parmi: octyle, isooctyle, décyle, isodécyle, cyclodécyle, dodécyle, cyclododécyle, isononyle, undécyle, lauryle, t-butylcyclohexyle, stéaryle; éthyl-2-hexyle;

- (iv) les éthers de vinyle de formule $R_6O\text{-}CH=CH_2$ ou les esters de vinyle de formule : $R_6\text{-}COO\text{-}CH=CH_2$

dans laquelle $R_6$ représente un groupe alkyle linéaire ou ramifié, comprenant de 8 à 22 atomes de carbone;

- (v) les macromonomères carbonés ayant au moins un groupe terminal polymérisable, obtenus à partir de monomères solubles au sens ci-dessus définis.

  Il s'agit de tout polymère, notamment oligomère, comportant sur une seule de ses extrémités un groupe terminal, notamment polymérisable, apte à réagir lors de la réaction de polymérisation avec les monomères considérés, pour former les chaînes latérales du polymère; ledit groupe terminal peut être avantageusement un groupe à insaturation éthylénique susceptible de se polymériser par voie radicalaire avec les monomères constituant le squelette. Ledit macromonomère permet de former les chaînes latérales du copolymère. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate.

  Parmi les macromonomères susceptibles d'être employés, on peut notamment citer, seuls ou en mélange, ainsi que leurs sels, :

- (v)a) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly(méthacrylate de stéaryle) à extrémité mono(méth)acrylate. De tels macromonomères sont notamment décrits dans les brevets EP895467 et EP96459.

- (v)b) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène. De tels macromonomères sont en particulier décrits dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

- (vi) les hydrocarbures éthyléniques ayant 2 à 10 carbones, tels que l'éthylène, l'isoprène, ou le butadiène.

De préférence, les monomères solubles sont choisis parmi :

- les (méth)acrylates de formule $CH_2$=CHCOOR ou $CH_2$=C($CH_3$)COOR dans laquelle R représente un groupe alkyle linéaire ou ramifié, comprenant 8 à 30 atomes de carbone ou un groupe cycloalkyle en C8 à C12;

- les homopolymères et les copolymères (méth)acrylate d'alkyle linéaire ou ramifié en C8-C22, présentant un groupe terminal polymérisable choisi parmi les groupes vinyle ou (méth)acrylate ayant au moins un groupe terminal polymérisable;

- les polyoléfines ayant un groupe terminal à insaturation éthylénique, en particulier ceux ayant un groupement terminal (méth)acrylate.

  On peut tout particulièrement citer comme monomère soluble, l'acrylate d'isobornyle, le méthacrylate d'isobornyle, l'acrylate d'éthyl-2-hexyle, le méthacrylate d'éthyl-2-hexyle, l'acrylate de lauryle, le méthacrylate de lauryle, l'acrylate de stéaryle, le méthacrylate de stéaryle, le méthacrylate de tertiobutyle, l'acrylate d'isobutyle; le méthacrylate de béhényle, l'acrylate de béhényle; ainsi que les macromonomères de poly(acrylate d'éthyl-2-hexyle) à extrémité mono(méth)acrylate; les macromonomères de poly(acrylate de dodécyle) ou de poly(méthacrylate de dodécyle) à extrémité mono(méth)acrylate; les macromonomères de poly(méth)acrylate de stéaryle à extrémité mono(méth)acrylate; et les macromonomères éthylène/butylène et éthylène/propylène à groupement terminal réactif (méth)acrylate.

  Les monomères insolubles peuvent en particulier être choisis parmi les monomères suivants, seuls ou en mélange, ainsi que leurs sels;

- (i) les (méth)acrylates de formule $CH_2$=CHCOOR ou $CH_2$=C($CH_3$)COOR dans laquelle R représente :

  - un groupe alkyle linéaire ou ramifié, comprenant 1 à 6 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène ou un groupe alkyle linéaire ou ramifié, en C1 à C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène; à l'exclusion du méthacrylate de tertiobutyle et de l'acrylate d'isobutyle;

- un groupe cycloalkyle en C3 à C6, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C5 à C8 ou aralkyle en C6 à C10 (groupe alkyle en C1 à C5);
notamment R peut être un groupe méthyle, éthyle, propyle, isopropyle, n-butyle, isobutyle, tertiobutyle, hexyle, cyclohexyle, 2-hydroxyéthyle, 2-hydroxybutyle, 2-hydroxypropyle, méthoxyéthyle, éthoxyéthyle, méthoxypropyle, phényle, 2-phényl-éthyle, t-butylbenzyle, benzyle, furfurylméthyle ou tétrahydrofurfurylméthyle, méthoxypolyoxyéthylène (ou POE-méthyle); POE-béhényle, trifluoroéthyle; diméthylaminoéthyle, diéthylaminoéthyle, diméthylaminopropyle.

- (ii) les (méth)acrylamides de formule $CH_2$=$CHCONR_4R_5$ ou $CH_2$=$C(CH_3)CONR_4R_5$
dans laquelle R4 et R5, identiques ou différents, représentent un atome d'hydrogène ou un groupe alkyle, linéaire ou ramifié, comportant de 1 à 6 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes $-NR'_4R'_5$, où R' et R'5 identiques ou différents représentent un groupe alkyle en C1 à C4;
ou bien R4 représente un atome d'hydrogène et R5 représente un groupe 1,1-diméthyl-3-oxobutyle;
de préférence, R4 et R5 peuvent être choisis parmi : hydrogène, méthyle, éthyle, propyle, isopropyle, n-butyle, isobutyle, tertiobutyle, hexyle, isohexyle, cyclohexyle, diméthylaminoéthyle, diéthylaminoéthyle, diméthylaminopropyle;

- (iii) les monomères à insaturation(s) éthylénique(s) comprenant au moins une fonction acide carboxylique, phosphorique ou sulfonique, ou anhydride, comme par exemple l'acide acrylique et l'acide méthacrylique; l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;

- (iv) les éthers de vinyle de formule $R_6O$-$CH$=$CH_2$ ou les esters de vinyle de formule : $R_6$-$COO$-$CH$=$CH_2$
dans laquelle $R_6$ représente un groupe alkyle linéaire ou ramifié, comprenant de 1 à 6 atomes;

- (v) le styrène et ses dérivés, notamment tels que le méthylstyrène, le chlorostyrène ou le chlorométhylstyrène;

- (vi) les macromonomères siliconés et notamment les polydiméthylsiloxanes, à groupement terminal mono(méth) acrylate, tels que ceux de formule (IIa) suivante :

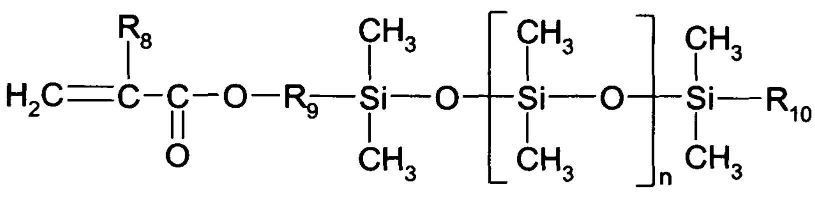

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de

5 à 100.
Comme macromonomères siliconés, on peut en particulier citer les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

- (vii) les monomères à insaturation(s) éthylénique(s) comprenant au moins une fonction amine tertiaire comme la 2-vinylpyridine, la 4-vinylpyridine;

- (viii) les composés vinyliques de formules : CH2=CH-R9, CH2=CH-CH2-R9 ou CH2=C(CH3)-CH2-R9

dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle);

- (ix) les monomères à insaturation éthylénique comprenant un ou plusieurs atomes de silicium tels que le méthacryloxypropyl triméthoxy silane, le méthacryloxypropyl tris(triméthylsiloxy)silane;

- (x) l'acrylate de perfluorooctyle.

De préférence, les monomères insolubles sont choisis parmi :

- les (méth)acrylates de formule $CH_2$=CHCOOR ou $CH_2$=C($CH_3$)COOR dans laquelle R représente un groupe alkyle linéaire ou ramifié, comprenant 1 à 6 atomes de carbone ou un groupe cycloalkyle en C3 à C6; à l'exclusion du méthacrylate de tertiobutyle et de l'acrylate d'isobutyle;
- les (méth)acrylamides de formule $CH_2$=CHCON$R_4R_5$ ou $CH_2$=C($CH_3$)CON$R_4R_5$
  dans laquelle R4 et R5, identiques ou différents, représentent un atome d'hydrogène ou un groupe alkyle, linéaire ou ramifié, comportant de 1 à 6 atomes de carbone; ou bien R4 représente un atome d'hydrogène et R5 représente un groupe 1,1-diméthyl-3-oxobutyle;
- les monomères à insaturation(s) éthylénique(s) comprenant au moins une fonction acide carboxylique,
- les monomères à insaturation éthylénique comprenant un ou plusieurs atomes de silicium tels que le méthacryloxypropyl triméthoxy silane, le méthacryloxypropyl tris(triméthylsiloxy)silane;
- l'acrylate de perfluorooctyle.

On peut tout particulièrement citer comme monomère insoluble, les (méth)acrylates de méthyle, d'éthyle, de propyle, de n-butyle, de cyclohexyle, de tertio-butyle; le méthacrylate d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle; le diméthylaminopropylméthacrylamide; l'acide (méth)acrylique; le styrène, l'acrylate de perfluorooctyle; leurs sels et leurs mélanges.
Préférentiellement, les monomères insolubles sont choisis parmi l'acrylate de méthyle, l'acrylate de méthoxyéthyle, le méthacrylate de méthyle, le méthacrylate d'isobutyle, le méthacrylate de 2-hydroxyéthyle, l'acide acrylique, l'acide méthacrylique, le méthacrylate de diméthylaminoéthyle, l'acrylate de perfluorooctyle, le méthacrylate de cyclohexyle, l'acrylate de tertio-butyle, leurs sels et leurs mélanges.
On préfère tout particulièrement les copolymères pour lesquels :

- le monomère soluble est choisi parmi l'acrylate d'isobornyle, le méthacrylate d'isobornyle, l'acrylate d'isobutyle, l'acrylate d'éthyle-2-hexyle et leurs mélanges; et/ou
- le monomère insoluble est choisi parmi le méthacrylate d'isobutyle et le méthacrylate de méthyle, et leurs mélanges.

Encore plus particulièrement, on préfère les copolymères comprenant :

- de l'acrylate d'isobomyle et du méthacrylate d'isobutyle;
- de l'acrylate d'isobomyle et du méthacrylate de méthyle;
- du méthacrylate d'isobomyle et du méthacrylate d'isobutyle;
- du méthacrylate d'isobomyle et du méthacrylate de méthyle;
- de l'acrylate d'isobornyle, de l'acrylate d'éthyle-2-hexyle; et du méthacrylate d'isobutyle;
- de l'acrylate d'isobornyle, de l'acrylate d'isobutyle, et du méthacrylate d'isobutyle;
- du méthacrylate d'isobornyle, de l'acrylate d'isobutyle, et du méthacrylate d'isobutyle et

- de l'acrylate d'isobornyle, de l'acrylate d'éthyle-2-hexyle, et du méthacrylate de méthyle.

Parmi les sels, on peut citer ceux obtenus par neutralisation des groupements acides à l'aide de base minérales telles que LiOH, NaOH, KOH, Ca(OH)2, NH40H ou Zn(OH)2; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.
On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Les monomères solubles tels que ci-dessus définis peuvent représenter 50 à 99% en poids du poids total de monomères, notamment de 60 à 90% en poids, voire de 70 à 85% en poids.
Les monomères insolubles tels que ci-dessus définis peuvent donc représenter 1 à 50% en poids du poids total de monomères, notamment de 10 à 40% en poids, voire de 15 à 30% en poids.

Le copolymère peut toutefois comprendre au moins un troisième monomère, voire un quatrième, et plus, qui est choisi parmi les monomères insolubles ou solubles, de préférence parmi les monomères solubles.

De préférence, le copolymère selon l'invention comprend au moins un monomère ayant une Tg inférieure ou égale à 20°C, notamment comprise entre - 150°C et 20°C, plus particulièrement comprise entre -130°C et 18°C, et encore mieux comprise entre -120°C et 15°C, ou un mélange de tels monomères.
Ce ou ces monomères peuvent être choisis parmi les monomères solubles ou insolubles ci-dessus mentionnés.
Ce ou ces monomères de Tg ≤ 20°C peuvent être présents à raison de 1 à 99% en poids, notamment 10-90% en poids, encore mieux 20-80% en poids, voire 25-75% en poids, par rapport au poids total du copolymère.

De préférence, le copolymère selon l'invention comprend donc également au moins un monomère ayant une Tg supérieure ou égale à 20°C, notamment comprise entre 25 et 150°C, plus particulièrement comprise entre 30 et 145°C, et encore mieux comprise entre 40 et 140°C, ou un mélange de tels monomères.
Ce ou ces monomères peuvent être choisis parmi les monomères solubles ou insolubles ci-dessus mentionnés.
Le ou les monomères de Tg ≥ 20°C peuvent donc être présents à raison de 1 à 99% en poids, notamment 10-90% en poids, encore mieux 20-80% en poids, voire 25-75% en poids, par rapport au poids total du copolymère.

Dans la présente description, on désignera par 'monomère de Tg' les monomères dont l'homopolymère a une telle Tg. Dans la présente invention, les Tg (ou température de transition vitreuse) sont des Tg théoriques déterminées à partir des Tg théoriques des monomères constitutifs de chacune des séquences, que l'on peut trouver dans un manuel de référence tel que le Polymer Handbook, 3$^{rd}$ ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i.

L'homme du métier saura choisir les monomères et leurs quantités en fonction du résultat recherché, en se basant sur ses connaissances générales, notamment sur la réactivité relative de chaque monomère. En particulier, il choisira les monomères et leur quantité, ainsi que le milieu solvant, de manière à obtenir un copolymère préférentiellement soluble dans ledit milieu solvant.

Les copolymères selon l'invention sont tout particulièrement solubles dans les milieux solvants lipophiles tels que les solvants notamment lipophiles et/ou les huiles carbonées usuellement employées en cosmétique.

On rappelle que par "soluble", on entend que le polymère ne forme pas de précipité dans le solvant. Avantageusement, le copolymère selon l'invention est soluble à une concentration d'au moins 1% en poids, dans l'isododécane, à 25°C, 1 atm.

Les copolymères à gradient selon l'invention peuvent être présents dans les compositions cosmétiques ou dermatologiques notamment topiques en une quantité de 0,1 à 95% en poids, de préférence 0,5 à 90% en poids, notamment 1 à 80% en poids, voire 5-70% en poids, et par exemple de 8 à 30% en poids, par rapport au poids total de la composition.

Les copolymères peuvent être présents dans la composition sous forme solubilisée, par exemple dans un

solvant organique ou une huile carbonée cosmétique.
On a constaté que les copolymères selon l'invention sont tout particulièrement solubles dans les solvants cosmétiques et qu'ils peuvent être mis en oeuvre dans les milieux cosmétiques organiques, tout en conservant des propriétés rhéologiques intéressantes.

Les compositions cosmétiques ou dermatologiques selon l'invention comprennent, outre lesdits copolymères, un milieu physiologiquement acceptable, notamment cosmétiquement ou dermatologiquement acceptable, c'est-à-dire un milieu compatible avec les matières kératiniques telles que la peau du visage
ou du corps, les lèvres, les cheveux, les cils, les sourcils et les ongles.

La composition peut avantageusement comprendre un milieux solvant qui peut être une phase grasse, qui peut elle-même comprendre des huiles et/ou des solvants de préférence lipophiles, ainsi que des corps gras solides à température ambiante tels que les cires, les corps gras pâteux, les gommes et leurs mélanges.

Parmi les constituants de la phase grasse, on peut préférentiellement citer les huiles et/ou solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(MPa)^{1/2}$.

Le paramètre de solubilité global δ selon l'espace de solubilité de Hansen est défini dans l'article "Solubility parameter values" de Eric A.Grulke de l'ouvrage "Polymer Handbook", 3éme édition, Chapitre VII, p.519-559 par la relation :

$$\delta = (dD^2 + dP^2 + dH^2)^{1/2}$$

dans laquelle

- dD caractérise les forces de dispersion de LONDON issues de la formation de dipôles induits lors des chocs moléculaires,
- dP caractérise les forces d'interactions de DEBYE entre dipôles permanents, et
- dH caractérise les forces d'interactions spécifiques (type liaisons hydrogène, acide/base, donneur/accepteur, etc.). La définition des solvants dans l'espace de solubilité selon Hansen est décrite dans l'article de C.M.Hansen "The three dimensional solubility parameters" J.Paint Technol. 39, 105 (1967).

Parmi les huiles et/ou solvants ayant un paramètre de solubilité global selon l'espace de solubilité de Hansen inférieur ou égal à 20 (MPa)1/2, on peut citer les huiles, volatiles ou non, qui peuvent être choisies parmi les huiles naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone ; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.
On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate
ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.
On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol.

On peut encore citer les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.

Parmi les composés volatils, on peut citer les huiles volatiles non siliconées, notamment les isoparaffines en C8-C16 comme l'isododécane, l'isodécane, l'isohexadécane et par exemple les huiles vendues sous les noms commerciaux d'ISOPAR, de PERMETHYL et notamment l'isododécane (PERMETHYL 99 A).

Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.

Ces huiles et/ou solvants peuvent être généralement présents en une teneur allant de 0,01 à 95%, de préférence de 0,1 à 90%, de préférence encore de 10 à 85% en poids, par rapport au poids total de la composition, et mieux de 30 à 80%.

La composition peut en outre comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30 % en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans

la composition, en une teneur allant de 0,01 à 50% en poids, par rapport au poids de la composition, de préférence de 0,01 à 30% en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphasé ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
La composition selon l'invention peut être une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.
La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.
Avantageusement, la composition selon l'invention peut être une composition de maquillage, notamment un fond de teint ou un rouge à lèvres.
L'invention a aussi pour objet un procédé cosmétique de maquillage ou de soin des matières kératiniques, notamment de la peau du corps ou du visage, des ongles, des cheveux et/ou des cils, comprenant l'application sur lesdites matières d'une composition cosmétique telle que définie précédemment.
En particulier, l'invention a pour objet un procédé cosmétique de maquillage de la peau du visage et/ou des lèvres, comprenant l'application sur lesdites matières d'une composition cosmétique de fond de teint ou de rouge à lèvres telles que définies précédemment.
L'invention est illustrée plus en détail dans les exemples suivants.

**Exemple 1 : préparation d'un copolymère à gradient de composition poly(acrylate d'isobornyle/acrylate de méthyle)**

Dans un tricol de 250 ml, on introduit 10 g d'isododécane, 21 g d'acrylate d'isobornyle, 9 g d'acrylate de méthyle, 1,338 g de iodo-perfluorohexane ($3.10^{-3}$ mole) et 0,308 g de Perkadox SE-10 ($9.10^{-4}$ mole). On mélange à 25°C et, après solubilisation, on plonge le ballon-tricol dans un bain d'huile thermostaté à 80°C. Après 1 h20 à 80°C, on observe un taux de conversion de 83%.
On obtient un copolymère à gradient de composition poly(acrylate d'isobornyle/acrylate de méthyle), avec un atome d'iode à l'extrémité de chaque chaîne. La masse molaire moyenne en nombre (Mn) obtenue est de 8800 g/mol (Mn théorique de 6500 g/mol).
Les fonctions iodées terminales peuvent être caractérisées par MALDI-TOF ou par RMN.

**Exemple 2 : préparation d'un copolymère à gradient de composition poly(acrylate d'isobornyle/acrylate de méthyle)**

Dans un tricol de 250 ml, on introduit 10 g d'isododécane, 24 g d'acrylate d'isobornyle, 6 g d'acrylate de méthyle, 1,339 g de iodo-perfluorohexane ($3.10^{-3}$ mole) et 0,308 g de Perkadox SE-10 ($9.10^{-4}$ mole). On mélange à 25°C et, après solubilisation, on plonge le ballon-tricol dans un bain d'huile thermostaté à 80°C. Après 1 h40 à 80°C, on observe un taux de conversion de 81%.
On obtient un copolymère linéaire et à gradient de composition poly(acrylate d'isobornyle/acrylate de méthyle), avec un atome d'iode à l'extrémité de chaque chaîne.
La masse molaire moyenne en nombre (Mn) obtenue est de 7650 g/mol (Mn théorique de 6300 g/mol).
Les fonctions iodées terminales peuvent être caractérisées par MALDI-TOF ou par RMN.

**Exemple 3 : préparation d'un copolymère à gradient de composition poly(acrylate d'isobornyle/acrylate de méthyle)**

Dans un tricol de 250 ml, on introduit 10 g d'isododécane, 15 g d'acrylate d'isobornyle, 15 g d'acrylate de méthyle, 1,339 g de iodo-perfluorohexane ($3.10^{-3}$ mole) et 0,308 g de Perkadox SE-10 ($9.10^{-4}$ mole). On mélange à 25°C et, après solubilisation, on plonge le ballon-tricol dans un bain d'huile thermostaté à 75°C. Après 1 h40 à 75°C, on observe un taux de conversion de 79%.
On obtient un copolymère à gradient de composition poly(acrylate d'isobornyle/acrylate de méthyle), avec un atome d'iode à l'extrémité de chaque chaîne. La masse molaire moyenne en nombre (Mn) obtenue est de 9500 g/mol (Mn

théorique de 6100 g/mol).
Les fonctions iodées terminales peuvent être caractérisées par MALDI-TOF ou par RMN.

**Exemple 4**

On prépare un fond de teint anhydre comprenant (% en poids) :

- cire de polyéthylène 12%
- huiles siliconées volatiles 25%
- phényltriméthicone 20%
- Microsphères de polyméthylméthacrylate 12%
- Polymère de l'exemple 1 6%
- Isododécane qsp 100%

Préparation :

On fait fondre les cires puis, quand tout est limpide, on ajoute la phényl triméthicone sous agitation, et les huiles de silicones; on ajoute ensuite les microsphères, l'isododécane et le polymère. On homogénéise pendant 15 minutes puis on coule la composition résultante que l'on laisse refroidir. On obtient un fond de teint anhydre.

**Exemple 5**

On prépare un stick de rouge à lèvres comprenant :

- Cire de polyéthylène 15 %
- Polymère de l'exemple 2 10 % en MA
- Polyisobutène hydrogéné (Parléam de Nippon Oil Fats) 25 %
- Pigments 10 %
- Isododécane qsp 100% La composition obtenue après application sur les lèvres présente de bonnes propriétés cosmétiques.

**Exemple 6 : Fond de teint E/H**

On prépare une composition de fond de teint comprenant les composés suivants :

Phase A

- Cetyl Dimethicone copolyol (ABIL EM 90 de GOLDSCHMIDT) 3 g
- Succinate d'isostéaryl diglycéryle (IMWITOR 780K de la société CONDEA) 0,6 g
- Isododécane 18,5 g
- Pigments (oxydes de fer de titane) 10 g
- Polymère de l'exemple 1 8,7 g en MSA
- Poudre de polyamide (NYLON-12) 8 g
- Parfum qs

Phase B

- Eau qsp 100 g
- Sulfate de magnésium 0,7 g
- Conservateur qs MSA : matière sèche active

La composition obtenue présente de bonnes propriétés cosmétiques.

**Exemple 7 : préparation d'un copolymère à bloc de composition polystyrène-b-poly(styrène/acrylate d'isobornyle)**

Dans un tube de 10 ml, on introduit 6 g de styrène, 0,0337 g de Perkadox SE-10 et 0,1467 g de iodo-perfluorohexane. Le tube est plongé dans un bain à 80°C pendant 3 heures. La conversion en styrène est de 95% et sa masse molaire moyenne en nombre (Mn) de 8345 g/mol (Ip = 2.16). On introduit alors dans le tube 3 g d'acrylate d'isobomyle avec 0,0168 g de Perkadox SE-10. Le tube est alors plongé dans un bain d'huile à 90°C pendant 4 heures. On obtient un copolymère à gradient de composition polystyrène-b-poly(styrène/acrylate d'isobornyle), avec un atome d'iode à l'extrémité de chaque chaîne.
Le copolymère obtenu a une masse molaire moyenne en nombre (Mn) de 16700 g/mol (Ip = 1.90).
L'observation en détection UV du déplacement du pic du copolymère final par rapport à celui du premier bloc prouve la formation d'un copolymère à bloc.
Les fonctions iodées terminales peuvent être caractérisées par MALDI-TOF ou par RMN.

## Revendications

1. Copolymère fonctionnalisé à au moins l'une de ses extrémités par un atome d'iode, et comprenant au moins deux monomères distincts choisis l'un parmi les monomères solubles dont l'homopolymère est soluble à une concentration d'au moins 3% en poids, dans l'isododécane, à 25°C, 1 atm., et l'autre parmi les monomères insolubles, dont l'homopolymère n'est pas soluble à une concentration d'au moins 3% en poids, dans l'isododécane, à 25°C, 1 atm.

2. Copolymère susceptible d'être obtenu par polymérisation radicalaire contrôlée par transfert dégénératif à l'atome d'iode :

   - d'un amorceur radicalaire,
   - d'un agent de transfert comprenant au moins un atome d'iode, et
   - d'au moins deux monomères distincts choisis l'un parmi les monomères solubles dont l'homopolymère est soluble à une concentration d'au moins 3% en poids, dans l'isododécane, à 25°C, 1 atm., et l'autre parmi les monomères insolubles, dont l'homopolymère n'est pas soluble à une concentration d'au moins 3% en poids, dans l'isododécane, à 25°C, 1 atm.

3. Copolymère selon l'une des revendications précédentes, dans lequel le monomère soluble est choisi parmi les monomères suivants, seuls ou en mélange, ainsi que leurs sels;

   - (i) les (méth)acrylates de formule $CH_2=CHCOOR$ ou $CH_2=C(CH_3)COOR$ dans laquelle R représente :

     - un groupe alkyle linéaire ou ramifié, comprenant 8 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques

     ou différents représentent l'hydrogène ou un groupe alkyle linéaire ou ramifié, en C1 à C6 ou un groupe phényle;

     - un groupe cycloalkyle en C8 à C12,
       notamment R peut être un groupe octyle, décyle, lauryle, isooctyle, isodécyle, dodécyle, t-butylcyclohexyle, stéaryle, éthyl-2-hexyle, isobornyle, béhényle;

   - (ii) le méthacrylate de tertiobutyle et l'acrylate d'isobutyle;

   - (iii) les (méth)acrylamides de formule $CH_2=CHCONR_4R_5$ ou $CH_2=C(CH_3)CONR_4R_5$ dans laquelle :

     - R4 représente un atome d'hydrogène ou un groupe alkyle, linéaire ou ramifié, comportant de 1 à 6 atomes de carbone;
       de préférence, R4 est choisi parmi: hydrogène, méthyle, éthyle, propyle, isopropyle, n-butyle, isobutyle, tertiobutyle, hexyle, isohexyle, cyclohexyle;

- R5 représente un groupe alkyle, linéaire ou ramifié, comportant de 8 à 18 atomes de carbone; de préférence, R5 est choisi parmi: octyle, isooctyle, décyle, isodécyle, cyclodécyle, dodécyle, cyclododécyle, isononyle, undécyle, lauryle, t-butylcyclohexyle, stéaryle; éthyl-2-hexyle;

- (iv) les éthers de vinyle de formule $R_6O-CH=CH_2$ ou les esters de vinyle de formule : $R_6-COO-CH=CH_2$ dans laquelle $R_6$ représente un groupe alkyle linéaire ou ramifié, comprenant de 8 à 22 atomes de carbone;

- (v) les macromonomères carbonés ayant au moins un groupe terminal polymérisable, obtenus à partir de monomères solubles au sens ci-dessus définis.

- (vi) les hydrocarbures éthyléniques ayant 2 à 10 carbones, tels que l'éthylène, l'isoprène, ou le butadiène.

**4.** Copolymère selon la revendication 3, dans lequel le macromonomère carboné est choisi parmi, seul ou en mélange, ainsi que leurs sels, :

- (v)a) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly(méthacrylate de stéaryle) à extrémité mono (méth)acrylate.
- (v)b) les polyoléfines ayant un groupe terminal à insaturation éthylénique, en particulier ceux ayant un groupement terminal (méth)acrylate, tels que les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly (éthylène/butylène)-polyisoprène.

**5.** Copolymère selon l'une des revendications précédentes, dans lequel le monomère soluble est choisi parmi :

- les (méth)acrylates de formule $CH_2=CHCOOR$ ou $CH_2=C(CH_3)COOR$ dans laquelle R représente un groupe alkyle linéaire ou ramifié, comprenant 8 à 30 atomes de carbone ou un groupe cycloalkyle en C8 à C12;
- les homopolymères et les copolymères (méth)acrylate d'alkyle linéaire ou ramifié en C8-C22, présentant un groupe terminal polymérisable choisi parmi les groupes vinyle ou (méth)acrylate ayant au moins un groupe terminal polymérisable;
- les polyoléfines ayant un groupe terminal à insaturation éthylénique, en particulier ceux ayant un groupement terminal (méth)acrylate.

**6.** Copolymère selon l'une des revendications précédentes, dans lequel le monomère soluble est choisi parmi l'acrylate d'isobornyle, le méthacrylate d'isobornyle, l'acrylate d'éthyl-2-hexyle, le méthacrylate d'éthyl-2-hexyle, l'acrylate de lauryle, le méthacrylate de lauryle, l'acrylate de stéaryle, le méthacrylate de stéaryle, le méthacrylate de tertiobutyle, l'acrylate d'isobutyle; le méthacrylate de béhényle, l'acrylate de béhényle; ainsi que les macromonomères de poly(acrylate d'éthyl-2-hexyle) à extrémité mono(méth)acrylate; les macromonomères de poly(acrylate de dodécyle) ou de poly(méthacrylate de dodécyle) à extrémité mono(méth)acrylate; les macromonomères de poly (méth)acrylate de stéaryle à extrémité mono(méth)acrylate; et les macromonomères éthylène/butylène et éthylène/propylène à groupement terminal réactif (méth)acrylate.

**7.** Copolymère selon l'une des revendications précédentes, dans lequel le monomère insoluble est choisi parmi les monomères suivants, seuls ou en mélange, ainsi que leurs sels;

- (i) les (méth)acrylates de formule $CH_2=CHCOOR$ ou $CH_2=C(CH_3)COOR$ dans laquelle R représente :

  - un groupe alkyle linéaire ou ramifié, comprenant 1 à 6 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques
  ou différents représentent l'hydrogène ou un groupe alkyle linéaire ou ramifié, en C1 à C6 ou un groupe

phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;
à l'exclusion du méthacrylate de tertiobutyle et de l'acrylate d'isobutyle;

- un groupe cycloalkyle en C3 à C6, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C5 à C8 ou aralkyle en C6 à C10 (groupe alkyle en C1 à C5);
notamment R peut être un groupe méthyle, éthyle, propyle, isopropyle, n-butyle, isobutyle, tertiobutyle, hexyle, cyclohexyle, 2-hydroxyéthyle, 2-hydroxybutyle, 2-hydroxypropyle, méthoxyéthyle, éthoxyéthyle, méthoxypropyle, phényle, 2-phényl-éthyle, t-butylbenzyle, benzyle, furfurylméthyle ou tétrahydrofurfurylméthyle, méthoxy-polyoxyéthylène (ou POE-méthyle); POE-béhényle, trifluoroéthyle; diméthylaminoéthyle, diéthylaminoéthyle, diméthylaminopropyle.

- (ii) les (méth)acrylamides de formule $CH_2=CHCONR_4R_5$ ou $CH_2=C(CH_3)CONR_4R_5$

dans laquelle R4 et R5, identiques ou différents, représentent un atome d'hydrogène ou un groupe alkyle, linéaire ou ramifié, comportant de 1 à 6 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes $-NR'_4R'_5$, où R' et R'5 identiques ou différents représentent un groupe alkyle en C1 à C4;
ou bien R4 représente un atome d'hydrogène et R5 représente un groupe 1,1-diméthyl-3-oxobutyle;
de préférence, R4 et R5 peuvent être choisis parmi : hydrogène, méthyle, éthyle, propyle, isopropyle, n-butyle, isobutyle, tertiobutyle, hexyle, isohexyle, cyclohexyle, diméthylaminoéthyle, diéthylaminoéthyle, diméthylaminopropyle;

- (iii) les monomères à insaturation(s) éthylénique(s) comprenant au moins une fonction acide carboxylique, phosphorique ou sulfonique, ou anhydride, comme par exemple l'acide acrylique et l'acide méthacrylique; l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;

- (iv) les éthers de vinyle de formule $R_6O-CH=CH_2$ ou les esters de vinyle de formule : $R_6-COO-CH=CH_2$ dans laquelle $R_6$ représente un groupe alkyle linéaire ou ramifié, comprenant de 1 à 6 atomes;

- (v) le styrène et ses dérivés, notamment tels que le méthylstyrène, le chlorostyrène ou le chlorométhylstyrène;

- (vi) les macromonomères siliconés et notamment les polydiméthylsiloxanes, à groupement terminal mono (méth)acrylate, tels que ceux de formule (IIa) suivante :

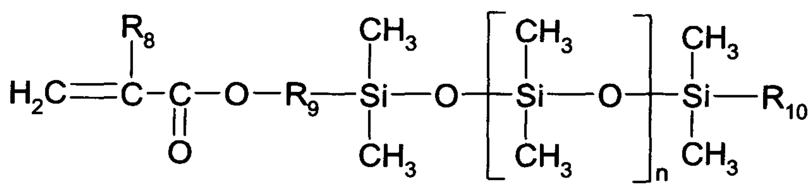

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

- (vii) les monomères à insaturation(s) éthylénique(s) comprenant au moins une fonction amine tertiaire comme la 2-vinylpyridine, la 4-vinylpyridine;

- (viii) les composés vinyliques de formules : CH2=CH-R9, CH2=CH-CH2-R9 ou CH2=C(CH3)-CH2-R9

dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle);

- (ix) les monomères à insaturation éthylénique comprenant un ou plusieurs atomes de silicium tels que le méthacryloxypropyl triméthoxy silane, le méthacryloxypropyl tris(triméthylsiloxy)silane;

- (x) l'acrylate de perfluorooctyle.

**8.** Copolymère selon l'une des revendications précédentes, dans lequel le monomère insoluble est choisi parmi :

- les (méth)acrylates de formule $CH_2$=CHCOOR ou $CH_2$=C($CH_3$)COOR dans laquelle R représente un groupe alkyle linéaire ou ramifié, comprenant 1 à 6 atomes de carbone ou un groupe cycloalkyle en C3 à C6; à l'exclusion du méthacrylate de tertiobutyle et de l'acrylate d'isobutyle;
- les (méth)acrylamides de formule $CH_2$=CHCON$R_4R_5$ ou $CH_2$=C($CH_3$)CON$R_4R_5$ dans laquelle R4 et R5, identiques ou différents, représentent un atome d'hydrogène ou un groupe alkyle, linéaire ou ramifié, comportant de 1 à 6 atomes de carbone; ou bien R4 représente un atome d'hydrogène et R5 représente un groupe 1,1-diméthyl-3-oxobutyle;
- les monomères à insaturation(s) éthylénique(s) comprenant au moins une fonction acide carboxylique,
- les monomères à insaturation éthylénique comprenant un ou plusieurs atomes de silicium tels que le méthacryloxypropyl triméthoxy silane, le méthacryloxypropyl tris(triméthylsiloxy)silane;
- l'acrylate de perfluorooctyle.

**9.** Copolymère selon l'une des revendications précédentes, dans lequel le monomère insoluble est choisi parmi les (méth)acrylates de méthyle, d'éthyle, de propyle, de n-butyle, de cyclohexyle, de tertio-butyle; le méthacrylate d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle; le diméthylaminopropylméthacrylamide; l'acide (méth)acrylique; le styrène, l'acrylate de perfluorooctyle; leurs sels et leurs mélanges.

**10.** Copolymère selon l'une des revendications précédentes, dans lequel le monomère insoluble est choisi parmi l'acrylate de méthyle, l'acrylate de méthoxyéthyle, le méthacrylate de méthyle, le méthacrylate d'isobutyle, le méthacrylate de 2-hydroxyéthyle, l'acide acrylique, l'acide méthacrylique, le méthacrylate de diméthylaminoéthyle, l'acrylate de perfluorooctyle, le méthacrylate de cyclohexyle, l'acrylate de tertio-butyle, leurs sels et leurs mélanges.

**11.** Copolymère selon l'une des revendications précédentes, dans lequel :

- le monomère soluble est choisi parmi l'acrylate d'isobornyle, le méthacrylate d'isobornyle, l'acrylate d'isobutyle, l'acrylate d'éthyle-2-hexyle et leurs mélanges; et/ou
- le monomère insoluble est choisi parmi le méthacrylate d'isobutyle et le méthacrylate de méthyle, et leurs mélanges.

**12.** Copolymère selon l'une des revendications précédentes, dans lequel le ou les monomère(s) soluble(s) représente 50 à 99% en poids du poids total de monomères, notamment de 60 à 90% en poids, voire de 70 à 85% en poids; et le ou les monomère(s) insoluble(s) représente 1 à 50% en poids du poids total de monomères, notamment de 10 à 40% en poids, voire de 15 à 30% en poids.

**13.** Copolymère selon l'une des revendications précédentes, comprenant au moins un monomère ayant une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères, qui peut être présent à raison de 1 à 99% en poids, notamment 10-90% en poids, encore mieux 20-80% en poids, voire 25-75% en poids, par rapport au poids total du copolymère.

**14.** Copolymère selon l'une des revendications précédentes, comprenant au moins un monomère ayant une Tg supérieure ou égale à 20°C, notamment comprise entre 25 et 150°C, plus particulièrement comprise entre 30 et 145°C, et encore mieux comprise entre 40 et 140°C, ou un mélange de tels monomères, qui peut être présent à raison de 1 à 99% en poids, notamment 10-90% en poids, encore mieux 20-80% en poids, voire 25-75% en poids, par rapport au poids total du copolymère.

**15.** Copolymère selon l'une des revendications précédentes, soluble à une concentration d'au moins 3% en poids, dans l'isododécane, à 25°C, 1 atm., de préférence à une concentration d'au moins 5% en poids, voire d'au moins 10% en poids.

**16.** Copolymère selon l'une des revendications précédentes, se présentant sous forme de polymères séquencés (ou polymère blocs) ou sous forme de polymères à gradient.

**17.** Copolymère selon l'une des revendications précédentes, ayant une structure de chaîne linéaire, greffée ou en étoiles.

**18.** Copolymère selon l'une des revendications précédentes, ayant une masse moléculaire moyenne en nombre comprise entre 2000 g/mol et 1 000 000 g/mol, notamment entre 3000 g/mol et 500 000 g/mol, et encore mieux entre 4000 g/mol et 200 000 g/mol, voire 5000 g/mol et 50 000 g/mol.

**19.** Copolymère selon l'une des revendications 2 à 18, pour lequel l'amorceur de polymérisation est choisi parmi les composés de type azoïque, notamment l'azobisisobutyronitrile; ou de type peroxyde tels que les hydroperoxydes ou les peroxydes organiques ayant 6-30 atomes de carbone, notamment le peroxyde de benzoyle ou de didécanoyle; ou encore des couples rédox, des peresters, des percarbonates ou des persulfates; ou le 2,5-bis(2-ethylhexanoylperoxy)-2,5dimethylhexane ou le tert-butyl peroxy-2-ethylhexanoate.

**20.** Copolymère selon l'une des revendications 2 à 19, pour lequel l'agent de transfert est représenté par la formule R-I, dans laquelle R est un radical alkyle, linéaire, ramifié ou cyclique, saturé ou insaturé, comprenant 1 à 30 atomes de carbone, et pouvant comprendre en outre 1 à 4 atomes d'iode supplémentaires, et/ou un ou plusieurs atomes de fluor, et/ou une ou plusieurs fonctions choisies parmi CN, COOH.

**21.** Copolymère selon la revendication 20, pour lequel R est choisi parmi les alkyles ayant 1 à 6 atomes de carbone, comprenant éventuellement un ou plusieurs atomes d'halogène, notamment de fluor et/ou une fonction CN.

**22.** Copolymère selon la revendication 20, pour lequel l'agent de transfert est choisi parmi le iodo-1-perfluorohexane, l'iodoacétonitrile ($ICH_2$-CN), le iodo-1-méthane, le diiodo-méthane, l'iodoforme ou triiodo-méthane, l'iodo-1-perfluoroisopropane, le diiodoperfluorohexane, l'iodo-1-phenylethane, l'iodo-1-propane, l'iodo-1-isopropane, l'iodo-1-phényle, le 1,4-diiodo-phényle et l'iodo-1-tertio-butane.

**23.** Copolymère selon l'une des revendications 2 à 19, pour lequel l'agent de transfert est macromoléculaire et se présente sous forme d'un polymère, homopolymère ou copolymère, fonctionnalisé par au moins un atome d'iode.

**24.** Composition cosmétique ou dermatologique comprenant, dans un milieu physiologiquement acceptable, au moins un copolymère tel que défini à l'une des revendications 1 à 23.

**25.** Composition selon la revendication 24, dans laquelle le copolymère est présent en une quantité de 0,1 à 95% en poids, de préférence 0,5 à 90% en poids, notamment 1 à 80% en poids, voire 5-70% en poids, et par exemple de 8 à 30% en poids, par rapport au poids total de la composition.

**26.** Composition selon l'une des revendications 24 à 25, comprenant une phase grasse, qui peut comprendre des huiles et/ou des solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(MPa)^{1/2}$.

**27.** Composition selon la revendication 26, comprenant au moins un constituant choisi parmi les huiles, volatiles ou non, naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone ; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.

**28.** Composition selon la revendication 27, comprenant au moins une huile choisie parmi les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides
ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges; le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol; les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges; les huiles volatiles non siliconées, notamment les isoparaffines en C8-C16 comme l'isododécane, l'isodécane, l'isohexadécane.

**29.** Composition selon la revendication 27, comprenant au moins un constituant choisi parmi les alcanes liquides à température ambiante, volatils ou non, et plus particulièrement le décane, l'heptane, le dodécane, l'isododécane, l'isohexadécane, le cyclohexane, l'isodécane, et leurs mélanges.

**30.** Composition selon l'une des revendications 26 à 29, dans laquelle ces huiles et/ou solvants sont présents en une teneur allant de 0,01 à 95%, de préférence de 0,1 à 90%, de préférence encore de 10 à 85% en poids, par rapport au poids total de la composition, et mieux de 30 à 80%.

**31.** Composition selon l'une des revendications 24 à 30, comprenant en outre au moins un constituant choisi parmi l'eau, les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles; les cires et/ou les gommes; les matières colorantes choisies parmi les colorants hydrosolubles, les colorants liposolubles, et les matières colorantes pulvérulentes comme les pigments, les nacres et les paillettes; les charges; les polymères additionnels notamment filmogènes; les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

**32.** Composition selon l'une des revendications 24 à 31, se présentant sous forme de suspension, de dispersion, de solution notamment organique, de gel, d'émulsion, notamment émulsion huile-dans-eau (H/E) ou eau-dans-huile (E/H),
ou multiple (E/H/E ou polyol/H/E ou H/E/H), sous forme de crème, de pâte, de mousse, de dispersion de vésicules notamment de lipides ioniques ou non, de lotion biphasé ou multiphase, de spray, de poudre, de pâte, notamment de pâte souple ; sous forme anhydre.

**33.** Composition selon l'une des revendications 24 à 32, se présentant sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cemes; un blush, un mascara, un eye-liner; un produit de maquillage des sourcils, un crayon à lèvres
ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel; d'une composition capillaire, notamment pour le maintien de la coiffure ou la mise en forme des cheveux.

**34.** Composition selon l'une des revendications 24 à 33, se présentant sous forme d'une composition de maquillage, notamment un fond de teint ou un rouge à lèvres.

**35.** Procédé cosmétique de maquillage ou de soin des matières kératiniques, notamment de la peau du corps ou du visage, des lèvres, des ongles, des cheveux et/ou des cils, comprenant l'application sur lesdites matières d'une composition cosmétique définie à l'une des revendications 24 à 34.

**36.** Procédé cosmétique de maquillage de la peau du visage et/ou des lèvres, comprenant l'application sur lesdites matières d'une composition cosmétique de fond de teint ou de rouge à lèvres telle que définie à l'une des revendications 24 à 34.

Office européen des brevets

# RAPPORT DE RECHERCHE EUROPEENNE

Numéro de la demande
EP 05 29 1040

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | WO 99/20659 A (THE B.F.GOODRICH CO.) 29 avril 1999 (1999-04-29) * revendication 1 * ----- | 1 | C08F2/38 |
| A | EP 0 489 370 A (DAIKIN IND. LTD.) 10 juin 1992 (1992-06-10) * revendication 1 * ----- | 1 | |

DOMAINES TECHNIQUES RECHERCHES (Int.Cl.7)
C08F

Le présent rapport a été établi pour toutes les revendications

| Lieu de la recherche | Date d'achèvement de la recherche | Examinateur |
|---|---|---|
| La Haye | 10 août 2005 | Cauwenberg, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

1

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 1040

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-08-2005

| Document brevet cité au rapport de recherche | Date de publication | Membre(s) de la famille de brevet(s) | Date de publication |
|---|---|---|---|
| WO 9920659 A | 29-04-1999 | US 6143848 A | 07-11-2000 |
| | | AU 751355 B2 | 15-08-2002 |
| | | AU 9808098 A | 10-05-1999 |
| | | BR 9813096 A | 15-08-2000 |
| | | CA 2306640 A1 | 29-04-1999 |
| | | JP 2001520282 T | 30-10-2001 |
| | | NO 20002041 A | 20-06-2000 |
| | | WO 9920659 A1 | 29-04-1999 |
| | | US 6784256 B1 | 31-08-2004 |
| EP 0489370 A | 10-06-1992 | JP 2100278 C | 22-10-1996 |
| | | JP 4202303 A | 23-07-1992 |
| | | JP 8026087 B | 13-03-1996 |
| | | CN 1062736 A | 15-07-1992 |
| | | DE 69124583 D1 | 20-03-1997 |
| | | DE 69124583 T2 | 12-06-1997 |
| | | EP 0489370 A1 | 10-06-1992 |
| | | RU 2111974 C1 | 27-05-1998 |
| | | US 5439980 A | 08-08-1995 |

EPO FORM P0460

Pour tout renseignement concernant cette annexe : voir Journal Officiel de l'Office européen des brevets, No.12/82